# EUROPEAN PATENT SPECIFICATION

(11) **EP 0 525 273 B1**
(45) Date of publication and mention of the grant of the patent: **16.10.1996**
(21) Application number: 91500089.7
(22) Date of filing: 02.08.1991
(51) Int. Cl.: G01N 33/49, G01N 33/86, G01N 11/10

(54) **Process and apparatus for the determination of the coagulation time of blood and plasma**
Verfahren und Vorrichtung zur Bestimmung der Gerinnungszeit von Blut und Plasma
Procédé et dispositif pour déterminer le temps de coagulation du sang

(43) Date of publication of application: 03.02.1993
(73) Proprietor: GRUPO GRIFOLS, S.A., E-08150 Parets Del Valles, (Barcelona) (ES)
(72) Inventor: Martinell Gisper-Sauch, Enrique, E-08010 Barcelona (ES); Gasull Llampallas, Antonio, E-08912 Badalona, (Barcelona) (ES)
(74) Representative: Duran Moya, Luis-Alfonso

(56) References cited:
- DE-A- 3 540 661
- FR-A- 2 318 421
- PATENT ABSTRACTS OF JAPAN vol. 6, no. 59 (P-110)(937) 16 April 1982

## Description

One of the characteristics with a higher interest in the handling of blood and plasma for clinical purposes consists in the evaluation of the coagulation time.

Various processes and systems are known for this aim which are based on physical effects related to the formation of fibrine which is typical of the coagulation cascade.

Among the apparatus and systems already known, the optical and mechanical coagulation meters may be cited. Some of these systems comprise rotating bars, corresponding to optical type meters; other systems rely on a rotating ball with sensors based on the Hall effect, others are based on vibrating or oscillating webs combined with vibration sensors, other types are based on the variation of electrical conductivity between electrodes and finally, other systems rely on the measurement of the variations of capillary viscosity.

The automatized systems require in order to increase the velocity of the process to carry out a series of different measurements simultaneously. This may be carried out by the arrangement in parallel of a series of sensors or having the reaction wells to rapidly cross in front of a sole sensor.

In both cases, some limitations appear as to space or time in the practical embodiment of the process.

To find a solution to the above drawbacks, the process and apparatus of this invention are based on completely different and original principles, consisting basically in the determination of the moment in which the coagulation process is initiated, by evaluation of the variation of mechanical characteristics appearing in the fluid to be analyzed. Therefore, the principle is essentially of a mechanical nature, requiring a ball, bar or any other object which movement may be disturbed by the reaction. This change will be detected in form of the corresponding image by means of an image-taking camera, in order that the generated signal may be processed as a dynamic image with the detection, by means of a digitation process, of the moment in which the change in the movement of the mechanical object being displaced within the fluid to be analyzed takes place. The fluid preferably consists, as above stated, in plasma or blood.

The determination of the moment in which the coagulation initiates is carried out by means of a programmable computer in which a comparison will be made of the values of certain parameters which depend on the mechanical performance of the fluid being analyzed with a certain standard or pattern. Therefore, the camera for taking images of the process will be used only for the capture and relying of the generated signal to the computer.

Any parameter may be used for the determination of the moment in which coagulation starts, in order to compare its value with a certain model. Such parameter may be any parameter which depends on the variation of the mechanical characteristics of the fluid to be analyzed in the moment in which the changes in the coagulation appear, that is, the formation of fibrine. Preferably to be used will be certain parameters as angular speed, linear speed and the position, that is, the radius of rotation of the mechanical element related with the fluid being analyzed.

After experimental work, the inventors have found that the parameters which are more suitable to carry out the invention consist in the linear velocity and the radius of rotation, as the angular speed is practically maintained during all the process.

The advantages to be obtained with the detection system of the present invention consist mainly in that the detection system in itself may be separated from the place in which the reaction is carried out, requiring only a "point of vision" from which the image will be captured. This characteristic allows the gathering (for example on a rectangular flat surface) of the reaction wells or cavities, reducing in this way the space needed, as no individual detector for each reaction is necessary. At the same time, the new process will allow the processing of multiple reaction wells in parallel, without requiring the reactions to be simultaneous, as it is not needed that all reactions start at the same time, given the fact that the invention allows the separate processing of each of them. At the same time, as the detection is carried out by means of a programmable computer, it is very simple to proceed to the change of the detection parameters, which in extreme cases could be carried out by the computer itself in an "intelligent" manner.

Therefore, the invention permits to carry out the automatic detection of the occurence of coagulation with great capacity and in a short time in comparison with previously known systems.

The apparatus to carry out the process of the present invention will preferably comprise a plate for carrying the reaction wells or cavities, which could consist in a state of the art microtiter plate preferably made out of plastic material, having multiple wells or small cavities in which the reactions will be carried out. The external shape of the plate may be changed according to convenience. It may be simply rectangular, for example, with ten columns and ten rows of reaction cavities. Each of the wells or reaction cavities will be fitted with a ball, so that when the plate will be rotated without changing its general orientation, the resulting movement for the balls within each of the wells or cavities will be circular, with constant sense of turning.

Generally speaking, the turning of each of the balls will be produced by means of a common mechanical arrangement for all of the balls, so that there will be no need to act particularly on each of them.

Given the characteristics of the system of the invention, there will be the possibility of using discardable microtiter plates with a great quantity of reaction cavities. This will help in speeding the general process and to the reduction of the space needed.

For its better understanding, a prefered embodiment will be explained in the following with reference being made to the enclosed drawings, in which:

Figure 1 shows a block chart corresponding to the process of the present invention.

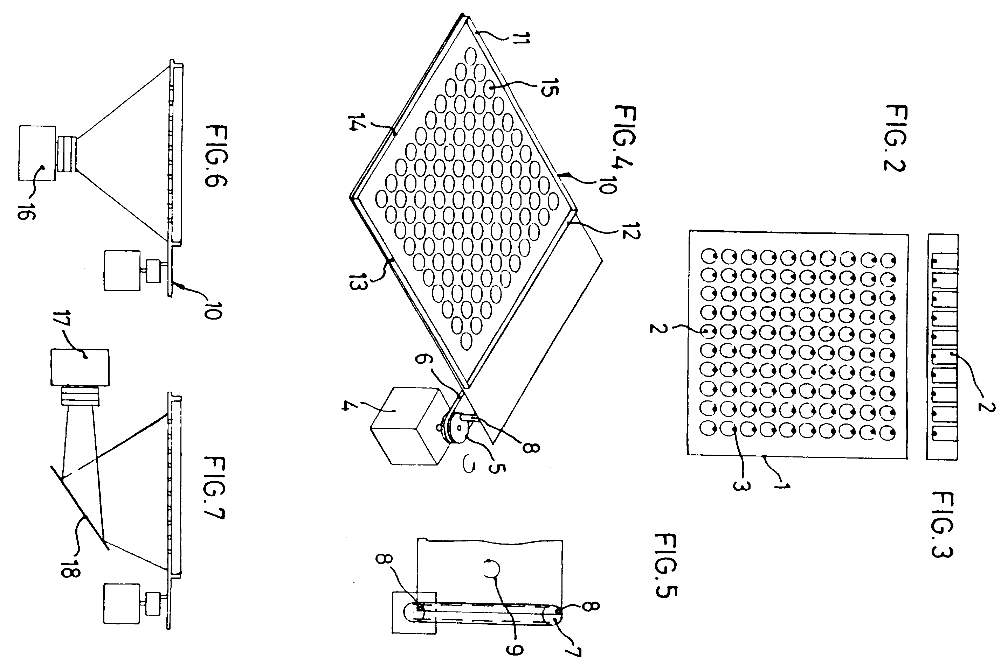

Figures 2 and 3 are a view from the top and a side view with a partial cross section of a sample carrying plate.

Figure 4 shows a pictorial view of the driving arrangement of the sample carrying plate.

Figure 5 shows a detail of the attachment of the driving motor to the sample carrying plate.

Figures 6 and 7 show examples of the arrangement of the image capturing camera.

Figure 8 is a simplified top view of the means to place the sample on the reaction cavities.

Figure 9 shows a simplified view of the means for feeding the reaction products to the wells of the cavity carrying plate according to the invention.

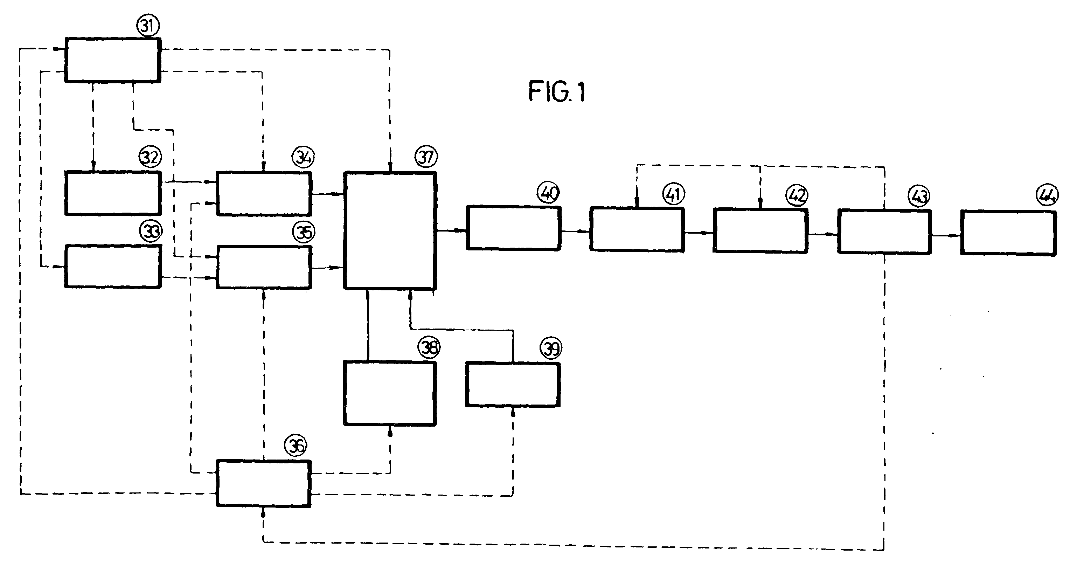

The process of the present invention comprises the arrangement of the elements shown in the block chart of figure 1, duly related among themselves so that a TV camera -40- may capture the images of plate -37- carrying the reaction cavities in which the different samples have been fed from a sample preparation device -34-. The feeding of the reaction products will be carried out by means of a proportioner device -35-. The sample preparing device -34- will receive sample -32- and the proportioner -35- will receive separately the reaction products -33-. Temperature will be controled by a temperature controling means -31- which will act on all elements under consideration, that is, the samples and sample preparing device, reaction products and proportioner device as well as the plate carrying the reaction cavities. The informations being captured by the TV camera -40-, will pass to data preparation step -41- to be relayed to an image processor -42-. Thereafter, the computer -43- will process the data to be fed to standard computer systems -44-.

A processor -36- will carry out the control of the process on the sample preparation device -34-, proportioner -35- and the system for the automatic feeding and discarding of plate -38- as well as the driving means -39- for the rotation of the plates carrying the reaction cavities. The system for the process control with receive the feed-back from the computer -43-.

The apparatus to carry out the process will comprise a microtiter plate -1- carrying a series of wells or cavities -2- for the samples distributed according to a pattern which usually will correspond to straight columns and rows as shown in the drawing or any other pattern adapted to the apparatus. Each of the cavities or wells will receive from one side a sample of the fluid to be analyzed, that is, of the blood or plasma which coagulation velocity is to be determined and from the other side it will receive the reaction products to be used to enhance coagulation.

In order that the apparatus may be suitable for carrying out the process of this invention, it will be essential that each cavity -2- has mechanical means to sense the physical variations of the fluid to be analyzed at the moment in which coagulation is initiated. In the drawings, a prefered embodiment has been shown in which each of the wells or cavities has one ball -3-.

The driving of plate -1- will be preferably carried out so that the mechanical means aimed at sensing the start of the coagulation may be subjected to a turning movement in only one direction. To this end, it is possible to have one driving motor -4- with a corresponding first pulley -5- which by means of a belt, is capable to drive a second end pulley -7- which has an excentrical shaft -8- articulated on platform -10- which supports the cavity carrying the microtiter plate -1-. In this way, the plate will have a circular movement in only one sense, so that in any point on the plate, the movement will correspond to turning in only one direction, as it has been shown by arrow -9- in figure 5.

For better timing of the movement of the platform and the cavity carrying plate supported on the platform, the belt -6- will be a toothed belt, the same as pulleys -5- and -7-.

Platform -10- receives the cavity carrying plate by means of rims -11-, -12-, -13- and -14-, shown only as an example. The platform has at the same time a series of holes -15- to allow the observation of cavity -2- of microtiter plate -1-from the bottom.

According to this invention, a TV camera is provided for capturing images. The TV camera will be located under the plateform, directed towards the upper part as in the version shown in figure 6, in which the camera has the reference numeral -16-. The camera could also be laterally located with the combination of a mirror, such as in the version shown in figure 7, in which the TV camera -17- captures the images through mirrors -18-.

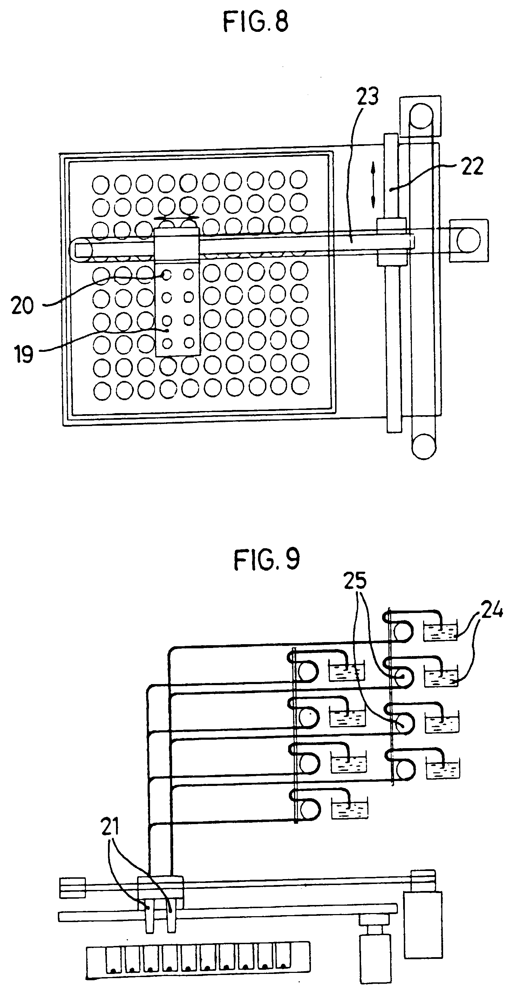

According to the present invention, the apparatus will be completed by means of a reactive preparation system which has been schematically shown in figures 8 and 9. The reactive preparation system is constituted by a support for a series of reactive despensing nozzles, each of which is connected to a peristaltic pump for the reactives by a pipe ending in the corresponding container.

The support has been shown schematically by means of plate -19- which bears multiple holes -20- for receiving the nozzles which have received reference numeral -21- in figure 9, and which movement may be obtained by a double arm system as shown by -22- and -23-. A series of containers -24- for the reactives will supply the reactives to nozzles -21- intermediate peristaltic pumps -25-shown in figure 9.

The driving system for plate -19- will have a movement according to three coordinated axis to obtain the necessary displacements in relation with the cavities of the plate, to allow the charging of the reactives on the same.

The TV camera will be connected to an image processing system composed by a capturing and digitation means and by signal processing means. The result will be the determination of the moment in which an alteration in the movement of each ball will appear.

The memory of the system will have a representation on real time of the image corresponding to the real view or scene. This information has to be processed by means of algorithms related to specific electronic circuits.

Complementarily, the apparatus of this invention will comprise means for the illumination of the scene, as well as for the thermostatic control of the plate carrying the reactives, as well as other auxiliary conventional means.

## Claims

1. Process for the determination of the coagulation time of blood and plasma of the type which comprises mechanical means contacting the fluid and the corresponding reactives to be analyzed, which mechanical means are given a displacement which is capable to be altered by the initiation of the coagulation of the fluid to be analyzed, characterized in that the images in real time of the mechanical means under displacement within the fluid to be analyzed are obtained by means of a television camera (40, 16, 17), being transmitted to a computer (43) which is capable to analyze the images to derive from the same the variation of one or more parameters related with the alteration of the movement of the mechanical means as a function of the initiation of the coagulation.

2. Apparatus for the determination of the coagulation time of blood and plasma according to the process of claim 1, having means to support a microtiter plate (1) with multiple cavities (2) for the samples and the reactives to be analyzed, the plate being transparent, to allow the optical observation, characterized in that it has a platform (10) to receive the microtiter plate (1) by means of rims (11), (12), (13) and (14), which platform has holes (15) for the observation of the cavities (2) of the plate (1) from the bottom, said platform (10) being driven so that it has a circular movement in only one direction in all and each of its points, the apparatus having a reactive feeding system with a number of nozzles (21) associated to a support which is driven in three dimensions to allow the charge of the various reactives in each of the sample containing wells (2) of the plate (1), the reactives being channeled by means of pipes from the corresponding containers (24) intermediate respective peristaltic pumps (25).

3. Apparatus according to claim 2, characterized in that the TV camera (16, 17) for capturing images in real time is attached to the apparatus in a form that its field of vision covers all of the sample cavities (2) in the support plate (1) from a unique point of vision, preferably from underneath of the sample carrying plate (1).

## Patentansprüche

1. Verfahren zur Bestimmung der Gerinnungszeit von Blut und Plasma, das eine mechanische Vorrichtung umfaßt, die das Fluid und die entsprechenden zu analysierenden Reaktanten berührt, wobei die mechanische Vorrichtung einer Verschiebung unterworfen ist, die durch den Beginn der Gerinnung des zu analysierenden Fluids verändert werden kann, dadurch gekennzeichnet, daß die Echtzeitbilder der in dem zu analysierenden Fluid verschobenen mechanischen Vorrichtung mittels einer Fernsehkamera (40, 16, 17) erhalten werden und an einen Rechner (43) übertragen werden, der die Bilder analysieren kann, um aus diesen die Veränderung eines oder mehrerer Parameter abzuleiten, die sich auf die Veränderung der Bewegung der mechanischen Vorrichtung als Funktion des Beginns der Gerinnung beziehen.

2. Vorrichtung zur Bestimmung der Gerinnungszeit von Blut und Plasma gemäß dem Verfahren nach Anspruch 1, mit einer Vorrichtung zum Tragen einer Mikrotitrationsplatte (1) mit mehreren Hohlräumen (2) für die zu analysierenden Proben und Reaktanten, wobei die Platte durchsichtig ist, um die visuelle Beobachtung zu ermöglichen, dadurch gekennzeichnet, daß die Vorrichtung eine Plattform (10) zum Aufnehmen der Mikrotitrationsplatte (1) mittels Rändern (11), (12), (13) und (14) besitzt, wobei die Plattform Löcher (15) für die Beobachtung der Hohlräume (2) der Platte (1) von der Unterseite her besitzt, die Plattform (10) so angetrieben wird, daß sie in allen ihren Punkten eine kreisförmige Bewegung in nur einer Richtung ausführt, die Vorrichtung ein Reaktantenzuführungssystem mit mehreren Düsen (21) besitzt, die einem Träger zugeordnet sind, der in drei Richtungen angetrieben wird, um das Einfüllen der verschiedenen Reaktanten in die jeweiligen Probengefäße (2) der Platte (1) zu ermöglichen, und die Reaktanten mittels Rohrleitungen aus entsprechenden Behältern (24) über entsprechende Schlauchpumpen (25) zugeführt werden.

3. Vorrichtung nach Anspruch 2, dadurch gekennzeichnet, daß die Fernsehkamera (16, 17) zum Aufnehmen der Echtzeitbilder derart an der Vorrichtung angebracht ist, daß ihr Sichtfeld alle Probenhohlräume (2) auf der Trägerplatte (1) von einem einzigen Sichtpunkt aus abdeckt, vorzugsweise von unterhalb der Probenträgerplatte (1).

## Revendications

1. Procédé de détermination du temps de coagulation du sang et du plasma, du type qui comprend des moyens mécaniques en contact avec le fluide et les réactifs correspondants à analyser, lesquels moyens mécaniques reçoivent un mouvement qui peut être modifié par le commencement de la coagulation du fluide à analyser,
caractérisé en ce que les images en temps réel des moyens mécaniques en mouvement dans le fluide à analyser sont obtenues au moyen d'une caméra de télévision (40, 16, 17), sont transmises à un ordinateur (43) qui est capable d'analyser les images pour déduire de celles-ci la variation d'un ou plusieurs paramètres reliés à la modification du mouvement des moyens mécaniques en fonction du commencement de la coagulation.

2. Appareil pour déterminer le temps de coagulation du sang et du plasma selon le procédé de la revendication 1, comprenant un moyen pour supporter une plaque de micro-titrage (1) avec de multiples alvéoles (2) destinés aux échantillons et aux réactifs à analyser, ladite plaque étant transparente pour permettre l'observation optique, caractérisé en ce qu'il comprend une plate-forme (10) pour recevoir la plaque de micro-titrage (1) à l'aide de rebords (11, 12, 13 et 14), laquelle plate-forme comporte des trous (15) permettant l'observation des alvéoles (2) de la plaque (1) depuis le dessous, ladite plate-forme (10) étant entraînée pour effectuer un mouvement circulaire dans une seule direction en chacun de tous ses points, l'appareil comportant un système d'alimentation en réactif avec un certain nombre de buses (21) associées à un support qui est entraîné dans les trois dimensions pour permettre le chargement des divers réactifs dans chaque alvéole (2) contenant les échantillons de la plaque (1), les réactifs étant canalisés au moyen de tubes provenant de récipients correspondants (24) grâce à des pompes péristaltiques respectives (25).

3. Appareil selon la revendication 2, caractérisé en ce que la caméra de télévision (16, 17) qui prend des images en temps réel est fixée à l'appareil de façon que son champ de vision couvre tous les alvéoles à échantillons (2) de la plaque de support (1) depuis un point d'observation unique, de préférence depuis le dessous de la plaque (1) de support d'échantillons.
